# EUROPÄISCHE PATENTSCHRIFT

(11) **EP 0 345 563 B1**
(45) Veröffentlichungstag und Bekanntmachung des Hinweises auf die Patenterteilung: **01.10.1997**
(21) Anmeldenummer: 89109515.0
(22) Anmeldetag: 26.05.1989
(51) Int. Cl.: G01N 33/00

(54) **Vorrichtung zum automatischen Kalibrieren von Gassensoren**
Apparatus for the automatic calibration of gas sensors
Appareil pour calibrer automatiquement des capteurs de gaz

(30) Priorität: 04.06.1988 DE 3819100
(43) Veröffentlichungstag der Anmeldung: 13.12.1989
(73) Patentinhaber: Endress + Hauser Conducta Gesellschaft für Mess- und Regeltechnik mbH + Co., D-70839 Gerlingen (DE)
(72) Erfinder: Schuldt, Hans-Peter, D-7257 Ditzingen 5 (DE)
(74) Vertreter: Otte, Peter, Dipl.-Ing.

(56) Entgegenhaltungen:
- EP-A- 242 946
- DE-A- 3 244 878
- US-A- 4 151 738
- US-A- 4 555 930

## Beschreibung

### Stand der Technik

Die Erfindung geht aus von einer Vorrichtung nach dem Oberbegriff des Anspruchs 1 und ist insbesondere für Gasdetektionssysteme geeignet, bei denen eine Vielzahl von Gassensoren tragenden Meßköpfen mit einer zentralen Auswerteelektronik verbunden sind.

Bei einer bekannten automatischen Kalibriervorrichtung (US-A-4 555 930) sind Sensoreinheiten mit einer Vor-Ort-Intelligenz ausgestattet und jeweils über Zweidrahtleitungen mit Untereinheiten verbunden, die ihrerseits mit anderen Untereinheiten sowie einer Hauptsteuereinrichtung verbunden sind.

Die Kommunikation zwischen den Sensoreinheiten, die nicht in einen speziellen Sensorkopf und an diese jeweils angeschlossene Sensoren aufgeteilt sind, erfolgt durch digitale Codezuordnung zu jeder Sensoreinheit, wobei auch eine automatische Kalibrierung möglich ist, die als interner Kalibrierzyklus abläuft und als Kalibrieranforderung entweder von der Hauptsteuereinheit oder zentralen Steuerwarte an die Untereinheiten ergeht oder auch von den einzelnen Sensoreinheiten eingeleitet werden kann. Bei Eingang eines solchen Kalibrieranforderungssignals schaltet die der jeweiligen Sensoreinheit zugeordnete Untereinheit ständig vorhandene Solenoidventile und veranlaßt aufeinanderfolgend die Zuführung eines Eichgases sowie eines Nullgases zur Sensoreinheit, wobei die sich dabei ergebenden Meßwerte von der zentralen Warte für spätere Benutzung gespeichert werden.

Vergleichbar mit diesem System ist eine Meldeeinrichtung für brennbare Gase (DE-A-32 44 878), bei der Signale in Digitalform von einem entfernten Meßfühler an ein Steuergerät übertragen und durch die Übertragungsleitung nicht beeinflußt werden. Eine entsprechende Zentraleinheit enthält auch einen Bedienungsteil, der einen Wählschalter aufweist, an welchem u. a. auch ein Kalibriervorgang angewählt werden kann, zusätzlich zu einer Tastenfeldmatrix, mit deren Hilfe die Werte des Prüfgases in den Speicher eingegeben werden. Es müssen daher zumindest vor einem Kalibriervorgang Bedienungspersonen im Bereich der zentralen Auswerteelektronik vorhanden sein, die Einstellungen am Bedienungsfeld ausführen.

Grundsätzlich ist bei Gasdetektionssystemen die vergleichsweise häufig notwendige Kalibrierung von Sensoren deshalb besonders umständlich, weil die Sensoren sich normalerweise in größerer Entfernung zu einer zentralen Warte oder Zentralelektronik befinden und eine Kalibrierung stets beim Austausch des Sensors, aber auch in sonstigen vorgegebenen Abständen wegen Alterung oder Umgebungseinflüssen, erforderlich wird.

So ist es auch bekannt, ein Kalibrierverfahren so durchzuführen, daß sich in der zentralen Meßwarte, in welcher auch die Auswerteelektronik vorhanden ist, eine erste Wartungsperson befindet, während eine zweite Wartungsperson mit Prüfgasflaschen entsprechender Konzentration vor Ort den jeweiligen Sensor mit den entsprechenden Prüfgasen beaufschlagt. Die Verständigung zwischen der Wartungsperson in der Zentrale und dem Mann vor Ort erfolgt über Gegensprech-Funkgeräte, wobei nach Beschickung eines jeweiligen Sensors mit dem Prüfgas in hinreichender Konzentration bis zur Erzielung eines Gleichgewichts dieser Umstand dann zusammen mit der Art des Prüfgases der Wartungsperson in der Zentrale mitgeteilt wird; diese nimmt dann an den entsprechenden Einstellpotentiometern in der Zentrale die Eichung vor, vorausgesetzt, daßnicht in der Zwischenzeit aus irgendwelchen Gründen eine Konzentrationsänderung aufgetreten ist. Ein solches Kalibrierverfahren ist langwierig und auch störanfällig, da eine 2-Mann-Kalibrierung notwendig ist. Kalibrierfehler treten daher häufig auf, nicht selten auch durch Verständigungsfehler verursacht.

Um diesen Schwierigkeiten zu begegnen und zu einer 1-Mann-Kalibrierung zu gelangen, ist es auch bekannt, zusammen mit den ohnehin schon erforderlichen separaten Verbindungskabeln zu jedem Meßkopf gleich noch eine Gasschlauchleitung zu diesem mitzuverlegen, die eine Austrittsöffnung im gassensitiven Bereich des Sensors aufweist und deren Verbindung mit der Prüfgasflasche in der zentralen Meßwarte hergestellt werden kann. Dies bedeutet allerdings, daß gegebenenfalls 500 m und möglicherweise noch viel längere Schlauchleitungen von der Meßwarte separat zu jedem Vor-Ort-Sensor verlegt werden müssen, mit dem enormen Nachteil, daß die Bedienungsperson in der zentralen Meßwarte eigentlich niemals genau weiß, wann das Prüfgas tatsächlich am Sensor angekommen ist, ob dort keine Störungen vorliegen und wann die Kalibrierung im Bereich der Einstellelemente der Meßwarte nun vorgenommen werden soll. Die Grundvorstellung bei dieser bekannten Kalibriermöglichkeit beruht jedenfalls darauf, daß man über die lange Schlauchleitung einen Gaspfropfen zum Sensor schickt, innerhalb welchem dann der vom Sensor zur Zentrale übermittelte Meßwert auf einen konstanten Wert einpendeln sollte, der dann als Maß für Nullpunkt bzw. Empfindlichkeit (Steigung) des Sensors als neuer Eichwert übernommen werden kann.

Erschwert werden diese Kalibrierprobleme noch durch den Umstand, daß die bekannten Gasdetektionssysteme kompliziert und umständlich ausgebildet sind. So umfaßt ein weiteres, auch für ausgedehntere Garagen oder Tunnel geeignetes System Gasentnahmeköpfe an verschiedenen Stellen, die über mechanische Schlauchverbindungen, die unter Umständen mehrere hundert Meter und darüber hinaus betragen müssen, mit einer gemeinsamen Auswertezentrale verbunden sind, wobei durch zyklisches, üblicherweise über angesteuerte Magnetventile bewirktes Umschalten von einem Schlauch zum anderen dem allerdings in der Zentrale dann nur einmal erforderlichen Sensor von den verschiedenen Entnahmestellen das dort vorhandene Gasgemisch zur Analyse zugeführt wird. Abgesehen von dem erheblichen mechanischen Aufwand, der nicht nur bei der Erstmontage, sondern auch nachfolgend zur Überwachung getrieben werden muß, beispielsweise um ein Abknicken von Schläuchen zu vermeiden, ist ein solches Meßverfahren auch besonders langwierig, weil sich durch die stets dazwischenliegende notwendige Spülung der Schläuche sehr hohe Totzeiten ergeben, bis der Gassensor wieder mit dem aktuellen Meßgas versorgt werden kann. Ein solches System benötigt eine Vielzahl mechanisch beweglicher Teile, eine besonders hohe Pumpenleistung, eine häufige Wartung und verbindet diese Umstände mit dem Nachteil, daß bei der Messung keine Redundanz auftritt, eine Selbstüberwachung des Systems ausgeschlossen ist und Zykluszeiten im Bereich bis zu 30 Minuten und darüber liegen können.

Es ist ferner bekannt, ein Gasdetektionssystem so aufzubauen, daß an den jeweils einer Überwachung oder Messung zu unterwerfenden Stellen jeweils ein mit einem Sensor bestückter Meßkopf angeordnet und über eine 5-adrige Leitung, gegebenenfalls auch nur über eine 3-adrige Leitung mit der Auswerteelektronik verbunden ist. Bei einer 5-adrigen Leitung dienen zwei der Leitungen zum Heizen des Sensors oder zu dessen Stromversorgung, zwei andere Leitungen dienen der Meßwertübertragung vom Meßkopf zur Auswerteelektronik und eine fünfte Leitung stellt die Masseverbindung her. Bei einer 3-adrigen Verbindung übernimmt die Masseleitung gleichzeitig auch die jeweils einen Verbindungsanschlüsse der beiden anderen Leitungsverbindungen und ersetzt immer eine Leitung. Ist an der gleichen Stelle oder, wie bei solchen Systemen üblicherweise beabsichtigt, an einer anderen Stelle eine weitere Gasanalyse erforderlich, dann müssen zur Detektion der zweiten Gaskomponente oder von Gasgemischen an anderen Stellen jeweils identische parallele Systeme mit Meßkopf und eigenem Verbindungskabel zur Auswerteelektronik geführt werden, d.h. die System- und Montagekosten sind erheblich.

Ähnliche, auch auf digitaler Basis ablaufende Kalibriervorgänge sind ferner bekannt aus der EP 0 242 946 A2. Eine Steuereinheit beaufschlagt über eine eine vorgegebene Zeitdauer bestimmende Intervallschaltung eine Timereinheit, die von der Zentrale aus eine dann vor Ort ablaufende Kalibrierung einleitet, die zunächst eine Reinigung des Meßsensors über eine spezielle Puffback-Einheit und anschließend die Zuführung von Nullgas und Kalibriergas umfaßt. Bei dieser Kalibriervorrichtung wird ebenfalls der gesamte Kalibriervorgang von der Zentrale und der an dieser vorhandenen Steuerlogik eingeleitet, wobei sich an den Meßsensoren keine Bedienungsperson befindet und von diesen auch keine den Kalibrierschritt einleitenden Signale herrühren, vergleichbar also mit dem einleitend erwähnten Gaserfassungssystem entsprechend US-A-4 555 930.

Der Erfindung liegt die Aufgabe zugrunde, eine automatische Kalibriervorrichtung für Gasdetektionssysteme mit mehreren an eine zentrale Auswerteelektronik angeschlossenen Meßköpfen derart auszubilden, daß nicht jede Sensoreinheit ihre eigene Kalibriervorrichtung mit eigener Nullgas- und Eichgaszufuhr hat, sondern daß eine einzige tragbare Kalibriervorrichtung von einer vor Ort befindlichen Bedienungspersons betätigt wird, die automatisch Signale zur Zentralauswerteelektronik schicken kann.

Diese Aufgabe wird durch ein automatisch kalibrierbares Gasanalysensystem mit den Merkmalen des Patentanspruchs 1 gelöst. Ein so ausgebildetes System hat den Vorteil, daß ohne manuellen Eingriff eine automatische Systemkalibrierung möglich ist, die einerseits dem Detektionssystem mitteilt, daß kalibriert wird (Durchführung einer Kalibrier-Vorkorrespondenz), die ferner in der Lage ist, automatisch das jeweilige Kalibriergas zu erkennen, die darüber hinaus den Kalibriervorgang überwacht und automatisch den Kalibrierwert, also den neuen Eichwert übernimmt und die darüber hinaus die Bedienungsperson ständig darüber informiert, wie weit der Kalibriervorgang durchgeführt und wann er abgeschlossen ist.

Dies enthält noch den weiteren besonderen Vorteil, daß die Bedienungsperson in den eigentlichen Kalibriervorgang nicht mehr einbezogen ist, also auch keine Kalibrierfehler mehr durch jede Art eines möglichen menschlichen Versagens, etwa Fehlinterpretation, falsches Einstellen u. dgl. auftreten können. Vorteilhaft ausgestaltet wird die durch die Erfindung ermöglichte Kalibrierung durch die Aufteilung der Vor-Ort-Apparatur in einen intelligenten, beispielsweise selbst mit einem Mikroprozessor ausgerüsteten Meßkopf mit Aufnahmemöglichkeiten für mindestens zwei Sensoren neben einem üblicherweise stets vorhandenen weiteren Temperatursensor und einem bidirektionalen digitalen Nachrichtenverkehr, also eine Digitalsignalübertragung zwischen der Elektronikzentrale und dem Meßkopf in beiden Richtungen, wodurch auch jede Störanfälligkeit in diesem Bereich beseitigt ist.

Dabei wird der Kalibriervorgang dadurch erleichtert, daß eine einzige Zweidrahtleitung eine im Grunde beliebige Anzahl von Vor-Ort-Apparaturen, jeweils bestehend aus Meßkopf mit einem oder mehreren Gassensoren und Temperatursensor mit der zentralen Auswerteelektronik in der Meßwarte verbindet, wobei auch beliebige Reihen/Parallelschaltungen der Meßköpfe möglich sind.

Vorteile sind hierbei die hohe Meßsicherheit, die geringe Wartung und die kostengünstige und einfache Installation, wobei noch besonders vorteilhaft ist, daß die Meßköpfe mit beliebigen Sensoren bestückt werden können, also entweder bei erforderlichem Austausch eines oder beider oder mehrerer Sensoren an einem Meßkopf wegen Alterung u. dgl. identische Sensoren wieder angebracht werden können, oder es können auch Sensoren für die Erfassung anderer Gase und/oder mit anderen Meßbereichen dem Standard- oder Universalmeßkopf zugeordnet werden, dennoch wird die Kalibrierung stets mit einwandfreier Identifizierung des betreffenden Sensors ablaufen. Der Grund hierfür liegt darin, daß zwei verschiedene Erkennungssysteme in einem solchen Gasdetektionssystem eingearbeitet sind, nämlich einmal eine Kennung zwischen den jeweiligen, an einen Meßkopf angeschlossenen Sensoren und die Adressierfähigkeit der Sensoren durch die Auswerteelektronik in der Zentrale, so daß die eine vorhandene Zweidrahtleitung problemlos für die Erfassung der Kalibrierdaten und Meßdaten einer Vielzahl von Vor-Ort-Apparaturen bei kürzesten Zykluszeiten durch die zentrale Auswerteelektronik eingesetzt werden kann.

Vorteilhaft ist die durch die Vor-Ort-Elektronik nunmehr ermöglichte automatische Systemkalibrierung in Verbindung mit einer speziellen Kalibrierkappe, wobei durch entsprechende Speicherung von Werten in der zentralen Auswerteelektronik, beispielsweise in einem EPROM, die zentrale Auswerteelektronik aus der Kennung des an den jeweiligen, adressierten Meßkopf angeschlossenen Sensors zunächst dessen Eigenschaften erfährt, also welche Gase er erfassen kann und welche Meßbereiche er aufweist. Diese Kennung wird in einer binären Verschlüsselung körperlich beim Einsetzen oder Anbringen des Sensors an den intelligenten Meßkopf von diesem erfaßt und auch für die Abfrage durch die zentrale Meßelektronik zur Verfügung gehalten, - durch das Kalibriersignal wird zusätzlich mitgeteilt, daß der betreffende Sensor kalibriert wird.

Durch die in den Unteransprüchen aufgeführten Maßnahmen sind vorteilhafte Weiterbildungen und Verbesserungen des im Hauptanspruch angegebenen Gasdetektionssystems möglich. Besonders vorteilhaft ist die Ausbildung der Kennung jedes Sensors in Form einer binären Verschlüsselung von Steckkontakten, wobei lediglich, je nach gewünschter Kennung im binären System, durch Verbindung von Lötpunkten Brücken hergestellt werden. Diese Kennung wird ab Werk vor Auslieferung angebracht, wodurch der beliebige Austausch vor Ort später möglich ist, da von der Intelligenz des Meßkopfes der zentralen Auswerteelektronik automatisch der Typ des Sensors sowie seines Meßbereichs gemeldet wird. Es ist dann möglich, auf einem geeigneten Anzeigedisplay in der zentralen Warte automatisch die richtige Konzentration und die jeweils gemessene Komponente anzuzeigen und die neuen Eichdaten zu übernehmen.

### Zeichnung

Ausführungsbeispiele der Erfindung sind in der Zeichnung dargestellt und werden in der nachfolgenden Beschreibung näher erläutert. Es zeigen:
- Fig. 1: schematisiert eine Kalibriereinrichtung, wie sie im Rahmen vorliegender Erfindung nunmehr möglich geworden ist;
- Fig. 2: schematisiert die verschiedenen Verbindungsmöglichkeiten zwischen den einzelnen Meßköpfen und der zentralen Auswerteelektronik.

### Beschreibung der Ausführungsbeispiele

Der Grundgedanke vorliegender Erfindung besteht darin, im Bereich der Gasanalyse durch die Möglichkeit einer Vor-Ort-Kommunikationsfähigkeit zwischen Sensor/Meßkopf einerseits und zentraler Auswerteelektronik andererseits eine automatische Kalibrierung einführen zu können, da die an den jeweiligen Sensor angebrachte und damit gleichzeitig an dessen Meßkopf herangebrachte Kalibriereinrichtung, nämlich im praktischen Fall die Kalibrierkappe, diese Kommunikationsfähigkeit ausnutzt und über den Meßkopf der zentralen Auswerteelektronik ein Kalibriersignal vermittelt. Durch eine Rückmeldung zum Meßkopf und der hierdurch bewirkten Möglichkeit der visuellen Kenntnisnahme durch die Bedienungsperson, daß die zentrale Auswerteelektronik die Kalibrierphase akzeptiert hat, weiß dann auch die Bedienungsperson, daß der Kalibriervorgang nunmehr abläuft. In Erägnzung wird der Bedienungsperson dann schließlich noch von der zentralen Auswerteelektronik das von dieser festgestellte Ende des Kalibriervorgangs mitgeteilt.

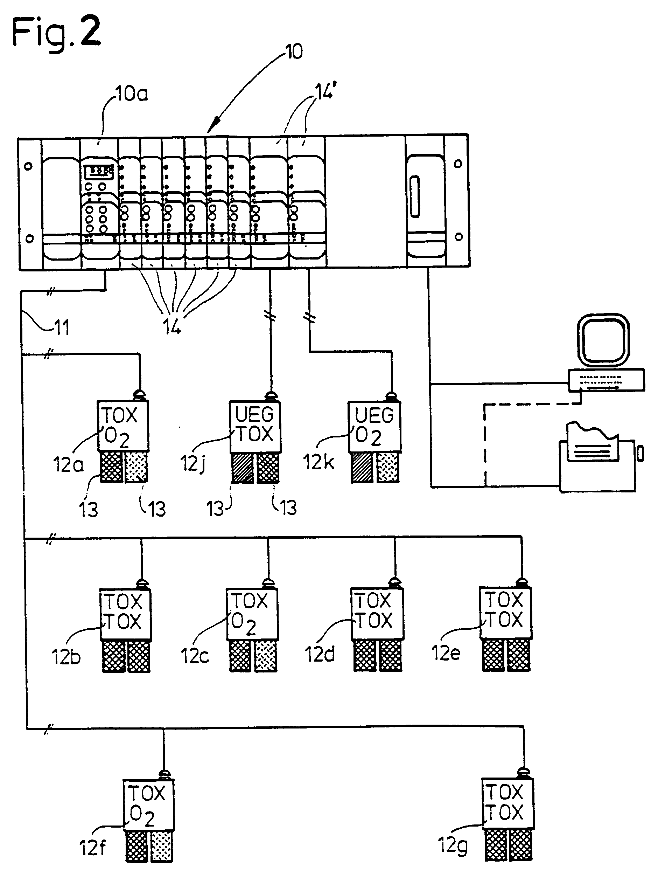

Zum besseren Verständnis vorliegender Erfindung wird zunächst auf ein bevorzugtes Ausführungsbeispiel eines Gasdetektionssystems anhand der Darstellung der Fig. 2 näher eingegangen, da sich hieraus auch die Möglichkeiten für den automatischen Kalibriervorgang besser erklären lassen; es wird aber darauf hingewiesen, daß die Erfindung nicht auf ein solches Gasdetektionssystem mit einer einzigen Zweidrahtleitung beschränkt ist, auf welcher die Kommunikation zwischen der Vielzahl der Meßköpfe und der zentralen Auswerteelektronik sowie die Stromversorgung der Meßköpfe abläuft, sondern bei beliebigen Gasdetektionssystemen eingesetzt werden kann, beispielsweise bei solchen, bei denen jeder einzelne Meßkopf (mit nur einem Sensor) über eine separate Leitung mit der Auswerteelektronik verbunden ist.

In Fig. 2 ist die zentrale Auswerteelektronik mit 10, eine von ihr ausgehende gemeinsame Zweidrahtleitung mit 11 und an diese Zweidrahtleitung angeschlossene Meßköpfe mit 12a, 12b ... bis 12g bezeichnet.

Jeder der Meßköpfe 12a bis 12k enthält einen eigenen Mikroprozessor und kann mit mehreren, bei dem dargestellten Ausführungsbeispiel mit zwei Sensoren unterschiedlichster Funktion bestückt werden zur Gasanalyse und zur Kalibrierung der Sensoren.

Die Sensoren sind in Fig. 2 durchgehend mit dem Bezugszeichen 13 versehen, unabhängig davon, welchen Typ sie darstellen oder welche Aufgaben (Meßbereich) sie erfüllen. Mit Vorzug stets ergänzend noch an jedem Meßkopf vorhandene Temperatursensoren sind der Übersichtlichkeit halber nicht dargestellt.

In der zentralen Auswerteelektronik ist eine Hauptrechenschaltung mit umfassenden Speichermöglichkeiten mit 10a im Einschubbereich bezeichnet; von dieser geht eine der Zweidrahtleitungen 11 aus, die mit den Meßköpfen 12a bis 12g verbunden ist. Diese Hauptrechenschaltung kann umfassend im Hardware-Bereich ausgebildet sein und verfügt für jeden der an sie angeschlossenen Meßköpfe 12a - 12g über entsprechende Informationen, so daß sie diese Meßköpfe einer gewünschten vorgegebenen Abfolge nach mit einem vorgegebenen Code adressiert, also anspricht, wodurch sich dann eine bidirektionale serielle Übertragung eines Protokolls anschließt. Jeder Meßkopf bildet mit Bezug auf die an ihn angeschlossenen Sensoren 13 eine Schnittstelle zur zentralen Auswerteelektronik; diese erhält vom entsprechenden Meßkopf beim normalen zyklischen Abfragedurchlauf sowie auch bei den später erläuterten Kalibrierungsschritten die jeweilige Kennung des Sensors S1 bzw. S2 für die angeschlossenen Gassensoren, die Meßwerte und sonstige Statusdaten übertragen.

Die Schnittstelle Meßkopf spricht mit der durchgegebenen Sensorkennung zunächst einen entsprechenden Speicherbereich in der Hauptrechenschaltung 10a der zentralen Auswerteelektronik an, so daß der anschließend, oder auch vorher, übermittelte jeweilige binärcodierte, also in digitaler Form über die Leitung übertragene Meßwert oder Kalibrierwert dieses Sensors die zutreffende Einordnung und Bewertung erfährt.

Im praktischen Aufbau sind an jedem Meßkopf (zunächst) 1 oder 2 Sensorgehäuse montiert (für die Gasanalyse, wobei natürlich je nach Auslegung dieses Schnittstellenbereichs, der Speichermöglichkeiten und der Ausgestaltung der zentralen Auswerteelektronik auch noch weitere Sensoren angeschlossen werden können, wenn hierzu Bedarf besteht). So können beispielsweise, um hier mit Zahlen zu arbeiten, etwa 10 ihrem Typ nach verschiedene Sensoren vorgesehen sein, mit zum Beispiel 30 verschiedenen Meßbereichen. Im Fehlerfall oder auch nach Wahl des Betreibers können sämtliche Sensoren ihrem Typ und ihrem Meßbereich nach problemlos an beliebigen Meßköpfen zur Umstellung auf andere Gaskomponenten oder andere Meßbereiche getauscht werden, wobei die Mikroprozessor-Elektronik im Meßkopf zusammen mit der digitalen Übertragung der Signale in bidirektionaler Richtung einen solchen Sensortausch durch die spezielle, sensorbezogene Kennung problemlos ermöglicht.

Die Sensoren werden über geeignete Sockelsteckkontakte am Meßkopf befestigt, wodurch gleichzeitig die elektrischen Verbindungsanschlüsse vorgenommen werden. Wie weiter vorn schon erwähnt, ist wesentlich, daß am Sockel bzw. Sensorbasisteil 19 die Kennung jedes Sensors für den Meßkopf lesbar angeordnet ist; diese sensorbezogene Kennung wird an dem jeweiligen Sensor sofort in der Produktion angebracht, so daß vor Ort dann jeder beliebige Austausch möglich ist, denn die Schnittstelle Meßkopf meldet durch die von ihr erfaßte Kennung der zentralen Auswerteelektronik automatisch den Typ des Sensors sowie dessen Meßbereich. Bei einem bevorzugten Ausführungsbeispiel eines Sensors kann die Kennung durch das Verbinden von elektrischen Kontaktpunkten durch Brükken im Sockelbereichvorgenommen werden; hierdurch kann der Mikroprozessor im Meßkopf die erforderlichen Angaben entnehmen und aus diesem Grund ist auch bei Nachbestückung ein Abgleich nicht mehr notwendig.

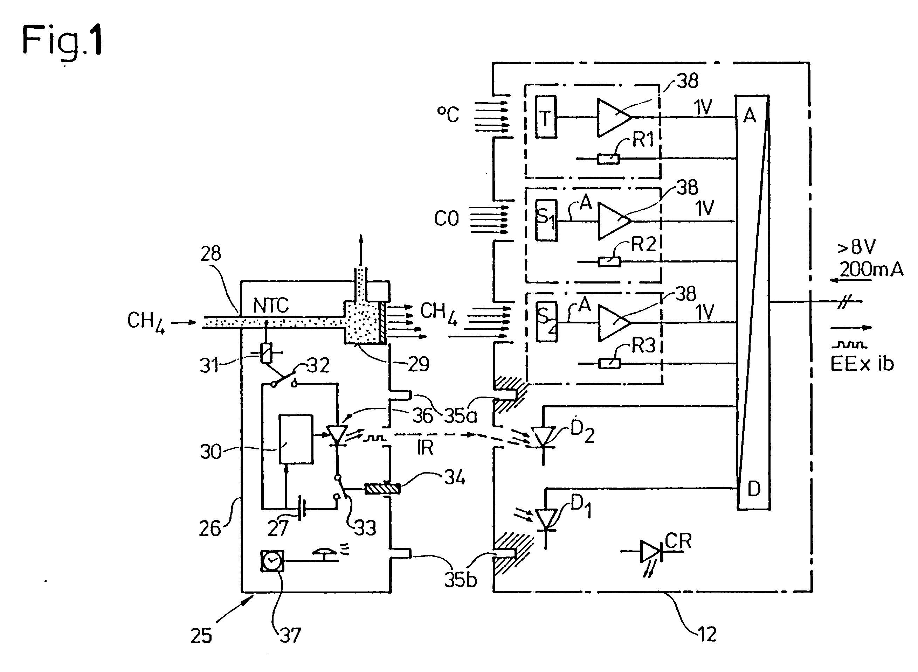

Entsprechend Fig. 1 umfaßt die Kalibriereinrichtung 25 in der praktischen Ausführungsform eine Kalibrierkappe 26, die über jedes mit dem Meßkopf fest verbunden bleibende Sensorgehäuse gestülpt wird. Die Kalibrierkappe 26 verfügt über eine eigene Stromversorgung wie beispielsweise eine Batterie 27, einen äußeren Gasanschluß 28, ein kappenförmiges Gehäuse, welches bei 29 angedeutet dargestellt ist, mit einer solchen Ausbildung und Form, daß es über den gassensitiven Bereich der Sensorbaugruppe (abgedichtet) gestülpt werden kann, und einen Elektronikbereich 30, der vom Meßkopf aufzunehmende Kalibriersignale erzeugt.

Gassensoren können nach Nullpunkt und Empfindlichkeit (Steigung) eine Kalibrierung erforderlich machen; entsprechend ist an den Gasanschluß 28 ein Prüfgas vorgegebener Konzentration jeweils anzuschließen. In der Leitung vom äußeren Gasanschluß 28 zur Kappe 29 befindet sich ein Strömungsmesser, vorzugsweise in Form eines NTC-Widerstands, der die vorhandene Eichgasströmung feststellt und, beispielsweise über ein zwischengeschaltetes Relais 31 einen ersten Schalter 32 betätigt. Durch das mechanische Aufstecken der Kalibrierkappe auf das Sensorgehäuse kann ein zweiter Schalter 33 ebenfalls betätigt werden, und zwar beispielsweise durch einen einfachen Schaltstift 34, der beim Aufstecken eingedrückt wird. Hier können natürlich auch andere Schaltmöglichkeiten vorgesehen sein, beispielsweise eine manuelle Schalterbetätigung durch die Bedienungsperson nach dem Aufstecken der Kalibrierkappe. Die Kalibrierkappe kann noch Führungsmittel 35a, 35b aufweisen zum ordnungsgemäßen Verbinden mit den Sensoren und dem Meßkopf, wobei, wie es sich versteht, auch die ansonsten vollautomatisch ablaufende Kalibrierung beider oder mehrerer Gassensoren am Meßkopf sowie auch des Temperatursensors möglich ist. In Fig. 1 sind die hier vorhandenen zwei Sensoren mit S1 und S2 sowie der Temperatursensor mit T bezeichnet.

Sind beide Schalter 32 und 33 geschlossen, dann erzeugt ein geeigneter Sender 36 im Bereich der Kalibrierkappe 25, vorzugsweise eine mit einem geeigneten binär-codierten Signal oder einfach mit einer vorgegebenen Frequenz (1 kHz) beaufschlagte Leuchtdiode ein Signal, beispielsweise dieses 1 kHz-Signal (im Infrarotbereich beispielsweise) und beaufschlagt hiermit eine Empfängerdiode D2 im Meßkopf, die für den Sensor S2 zuständig ist.

Liegt also die richtige Eichgasströmung vor, dann erzeugt die Kalibrierkappe das Kalibriersignal (zunächst für einen ersten Sensor S2); der Mikroprozessor des Meßkopfes 12 erkennt dieses Kalibriersignal und überträgt es als Kalibrierhauptsignal an die zentrale Auswerteelektronik, so daß die Auswerteelektronik in Korrespondenz mit diesem speziellen Meßkopf jetzt in eine "Kalibrierphase" übergeht, wobei die zentrale Auswerteelektronik mit einem Rücksignal an den Meßkopf zunächst die Erkennung und die Bereitschaft zur Kalibrierung meldet. Dieses Kalibrier-Rückbestätigungssignal wird am Meßkopf in geeigneter Weise angezeigt, und zwar über eine Leuchtdiode CR, was bedeutet, daß das Funktionssignal "Kalibrierung" nunmehr aufleuchtet und auch die Bedienungsperson weiß, daß das gesamte Prozessorsystem die Kalibrierwerte akzeptiert und übernimmt.

Hier ist noch auf etwas hinzuweisen: Da die zentrale Auswerteelektronik erkennen muß, ob es sich um ein sogenanntes Nullgas zur Nullpunktbestimmung bzw. um ein Prüfgas zur Empfindlichkeitsbestimmung handelt, interpretiert die zentrale Auswerteelektronik Gaskonzentrationen von z.B. weniger als 10%, die sie während der "Kalibrierphase" erfaßt, als Eich- oder Nullgas zur Nullpunktbestimmung und Konzentrationen über z.B. 20 % zur Empfindlichkeitsbestimmung, wobei das Prüfgas dann eine vorgegebene Konzentration von beispielsweise 50 % des Meßbereich-Endwertes haben kann. Aufgrund der in der zentralen Auswerteelektronik gespeicherten Werte und Daten ist diese über die Prüfgaszusammensetzung, mit denen sie konfrontiert wird, informiert und kann dann automatisch die entsprechenden Eichvorgänge durchführen.

Die Eichung läuft im Prinzip so ab, daß die zentrale Auswerteelektronik bei den während der Kalibrierphase eingehenden Meßwerten darauf achtet, wann das eingehende Signal seine Konzentration nicht mehr ändert, also das "Konzentrationssignal" in einer bestimmten Zeit gegen Null bzw. gegen einen sonstigen stabilen Zustand läuft. Dies erkennt die zentrale Auswerteelektronik als Hinweis darauf, daß der Sensor im eingeschwungenen Zustand die Prüfgaskonzentration übermittelt und sie korrigiert, gegebenenfalls entsprechend die in ihr gespeicherten Angaben für Nullpunkt und Steilheit (Empfindlichkeit) dieses betreffenden Sensors, wobei dann der Kalibriervorgang vorzugsweise nicht durch das Bedienungspersonal, sondern auf Weisung der zentralen Auswerteelektronik automatisch beendet wird. Die zentrale Auswerteelektronik nimmt nach Beendigung des Kalibriervorgangs das Funktionssignal "Kalibrierung" (Leuchten der Diode CR) wieder weg oder läßt diese intermittierend aufleuchten, so daß die Bedienungsperson den Kalibriervorgang für diesen Sensor als beendet erkennt und die Kalibrierkappe abziehen kann für die Kalibrierung des nächsten Sensors an diesem Meßkopf. Hierzu wird die Kalibrierkappe 25 auf diesen anderen Sensor aufgesteckt, das Kalibriersignal wird von der Kalibrierkappe erneut erzeugt und gelangt nunmehr auf einen zweiten Empfänger in Form einer Photodiode D1, wobei beispielsweise nunmehr eine Frequenz von 2 kHz erzeugt werden kann. Es ist aber auch möglich, dieses insofern dann unterschiedliche Kalibriersignal dem gleichen Empfänger im Meßkopf zuzuführen, wobei es auch möglich ist, die Kalibrierkappe sofort so auszubilden, daß sie über zwei angrenzende Kappen verfügt, die die jeweiligen Sensoren am Meßkopf aufeinanderfolgend mit den Prüfgasen beaufschlagen.

Die Kalibrierkappe kann auch über eine Schaltung 37 zur Erzeugung eines eigenen Zeittaktes verfügen. Ausdrücklich sei darauf hingewiesen, daß die Kalibrierung durch Einstellen entsprechender Schaltungen im Bereich des Meßkopfes bzw. entsprechender Speicherung der neuen, aus der Kalibrierung entstandenen Daten im Meßkopf durchgeführt werden kann; dies ist auch insofern vorteilhaft, als der Meßkopf ohnehin über lediglich durch das Verstärkerzeichen in Fig.3 angedeutete Elektronikmittel 38, zugeordnet jedem Sensor verfügt, um als Interface insofern standardisierte Meßsignalgrößen zwischen 0 und 1 V in binärcodierter Form auf die Leitung geben zu können.

Zusammengefaßt: Das Gasdetektionssystem im Meßkopf und zentraler Auswerteelektronik erkennt automatisch, daß kalibriert wird, wobei der Kalibriervorgang automatisch überwacht und die Kalibrierwerte automatisch übernommen werden mit ständiger Information der Bedienungsperson, so daß auch hier mit einem Schlage die Probleme bei der stets notwendigen Kalibrierung von Gassensoren, wie sie nach dem Stand der Technik auftreten, beseitigt sind. Die mit der Kalibrierung beauftragte Bedienungsperson braucht lediglich noch die Kalibrierkappe auf das Sensorgehäuse zu stecken und das jeweils zutreffende Prüfgas zuzuführen. Sämtliche anderen Vorgänge laufen automatisch aufgrund der bidirektionalen Kommunikationsfähigkeit des Systems ab, so daß Fehler vollkommen ausgeschlossen sind.

## Patentansprüche

1. Vorrichtung zum automatischen Kalibrieren von Gassensoren, insbesondere für Gasdetektionssysteme mit einer Vielzahl von an eine zentrale Auswerteelektronik angeschlossenen, Gassensoren tragenden Meßköpfen (12a bis 12g), die eine Vor-Ort-Elektronik enthalten und über eine Fernleitung (11) mit der zentralen Auswerteelektronik (10) verbunden sind, ferner mit Mitteln zur Aufleitung mindestens eines Prüfgases in den gassensitiven Bereich des jeweiligen Sensors, wobei dem diesen Sensor tragenden Meßkopf (12) bei Einleitung der Kalibrierphase ein Kalibriersignal zugeführt wird, und im Bereich der Auswerteelektronik ein Stellmittel zur Übernahme der neuen Eichwerte als Folge der automatisch ablaufenden Kalibrierphase angeordnet ist, dadurch gekennzeichnet, daß eine an dem Meßkopf (12) anordenbare Kalibriervorrichtung (25) vorgesehen ist, welche eine Kalibrierkappe (26) und Führungsmittel (35) zum Verbinden der Kalibrierkappe (26) mit den Sensoren des Meßkopfes (12) aufweist, daß die Kalibrierkappe (26) einen äußeren Gasanschluß (28) für ein Prüfgas, welches durch ein kappenförmiges Gehäuse zum Meßkopf (12) fließt, einen Elektronikbereich (30), welcher die von dem Meßkopf (12) aufzunehmenden Kalibriersignale erzeugt, einen Sender (36) , welcher das Kalibriersignal einer Empfangseinheit (D1, D2) in dem Meßkopf (12) zuführt, so daß daraufhin durch die mit dem jeweiligen Gassensor verbundene Vor-Ort-Elektronik des Meßkopfes (12) das Kalibrierhauptsignal zur Weiterleitung an die Auswerteelektronik (10) erzeugbar und hierdurch die automatisch ablaufende Kalibrierphase einleitbar ist, und eine Stromversorgung aufweist.

2. Vorrichtung nach Anspruch 1, dadurch gekennzeichnet, daß das Kalibriersignal der Empfangseinheit (D1, D2) in dem Meßkopf (12) dann zuführbar ist, wenn die Kalibrierkappe (26) sich in Eichposition befindet und das Prüfgas dem gassensitiven Element jedes Sensors zugeführt wird.

3. Vorrichtung nach Anspruch 1 oder 2, dadurch gekennzeichnet, daß die Kalibrierkappe (26) den Meßkopf (12) und dieser weiterführend die mit ihm verbundene zentrale Auswerteelektronik (10) mit einem spezifischen, den jeweiligen Sensor (S1, S2, T) betreffenden Kalibriersignal bzw. Kalibrierhauptsignal beaufschlagt.

4. Vorrichtung nach einem der Ansprüche 1 bis 3, dadurch gekennzeichnet, daß das Kalibriersignal ein mit vorgegebener Frequenz gepulstes optisches Signal, z.B. Infrarotsignal, ist, welches von einer Sendediode (36) erzeugt und einer Empfangsdiode (D1, D2) im Meßkopf dann zugeführt wird, wenn ein Strömungssensor in der Kalibrierkappe (26) das Vorhandensein des Prüfgases und ein Kontaktsensor (34) oder ein von der Bedienungsperson betätigter Schalter deren Anlage an den jeweiligen, zu kalibrierenden Sensor signalisieren.

5. Vorrichtung nach Anspruch 4, dadurch gekennzeichnet, daß der Strömungssensor ein NTC-Widerstand ist, der in Reihe mit dem die Anlage der Kalibrierkappe signalisierenden Kontaktsensor, siehe Schaltstift (34), liegt und die Sendediode (36) zur Angabe des Kalibriersignals aktiviert.

6. Vorrichtung nach einem der Ansprüche 1 bis 5, dadurch gekennzeichnet, daß die zentrale Auswerteelektronik (10) nach Empfang des Kalibriersignals und der Kennung des betreffenden Sensors ein Kalibrier-Bestätigungssignal zum jeweiligen Meßkopf (12) rückführt, daß der Meßkopf (12) daraufhin der Bedienungsperson ein Kalibrier-Betätigungssignal vermittelt und daß die zentrale Auswerteelektronik das Ende des Kalibriervorgangs automatisch durch Beeinflussung des Kalibrier-Bestätigungssignals anzeigt bei gleichzeitiger Übernahme des oder der neuen Kalibrierwerte.

7. Vorrichtung nach Anspruch 6, dadurch gekennzeichnet, daß die zentrale Auswerteelektronik (10) in der Kalibrierphase den vom jeweils mit dem Eichgas beaufschlagten Sensor eingehenden Meßwert auf konstantes Verhalten über der Zeit für einen vorgegebenen Zeitraum überprüft und die neuen Eichwerte bei einem Meßwert unterhalb einer vorgegebenen Schwelle, z.B. Nullgas, als Nullwert bzw. oberhalb einer vorgegebenen Schwelle als Steigungswert, z.B. Empfindlichkeit, vorgegebener Größe speichert und übernimmt.

8. Vorrichtung nach einem der Ansprüche 1 bis 7, dadurch gekennzeichnet, daß die zentrale Auswerteelektronik (10) mit einer Vielzahl von Meßköpfen (12; 12a, 12b...12g) vor Ort lediglich über eine einzige gemeinsame Zweidrahtleitung (11) verbunden ist, daß die Intelligenz bzw. der Mikroprozessor jedes Meßkopfes (12) vor Ort eine bidirektionale digitale Kommunikation mit der zentralen Auswerteelektronik ermöglicht und daß an jedem Meßkopf (12; 12a, 12b, 12c...12g) mindestens zwei Sensoren verschiedener Art oder unterschiedlicher Meßbereiche sowie ein zusätzlicher Temperatursensor angeschlossen sind.

9. Vorrichtung nach Anspruch 8, dadurch gekennzeichnet, daß in der Verbindung zwischen jeweiligem Sensor und Meßkopf eine den Sensor nach Typ, Meßgas und Meßbereich identifizierende Kennung auftritt, die vom Meßkopf erfaßt wird.

10. Vorrichtung nach Anspruch 9, dadurch gekennzeichnet, daß die mit den Meßköpfen (12, 12a bis 12g) zu verbindenden Sensoren (13) jeweils vereinheitlichte Sockelanschlüsse und die Meßköpfe entsprechend ausgebildete Aufnahmeöffnungen, z. B. Stecker und Buchse, aufweisen und daß die am jeweiligen Sensor angebrachte Kennung in Form von elektrischen Kontakten für den Meßkopf erkennbar ausgebildet ist.

11. Vorrichtung nach Anspruch 10, dadurch gekennzeichnet, daß am jeweiligen Sensor dessen Typ, Meßgas und Meßbereich in binär-codierter Form angebende Lötkontaktbrücken für die Meßkopf-Sockelfassung identifizierbar angeordnet sind.

12. Vorrichtung nach einem der Ansprüche 8 bis 11, dadurch gekennzeichnet, daß mindestens im Bereich der zentralen Auswerteelektronik Speichermittel vorgesehen sind mit bestimmten Sensoren nach Typ, Meßgas und Meßbereichen zugeordneten Daten derart, daß die Auswerteelektronik nach Empfang der Sensorkennung, entsprechenden Statussignalen, den jeweiligen Meßwerten und den Kalibrierungswerten diese auswerten und verarbeiten kann.

13. Vorrichtung nach einem der Ansprüche 8 bis 12, dadurch gekennzeichnet, daß in dem als EPROM ausgebildeten Speicher der zentralen Auswerteelektronik entsprechende Arbeitsroutinen je empfangener Sensorkennung gespeichert sind.

## Claims

1. Apparatus for the automatic calibration of gas sensors, in particular for gas detection systems, comprising a plurality of measuring heads (12a to 12g) connected to a central electronic evaluation unit and carrying gas sensors, the measuring heads (12a to 12g) containing an in situ electronic unit and being connected to the central electronic evaluation unit (10) via a transmission line (11) and also being connected to means for supplying at least one testing gas to the gas-sensitive region of the respective sensor, wherein a calibrating signal is sent to the measuring head (12) carrying this sensor on initiation of the calibration phase, and an adjusting means for adopting the new calibration values as a result of the automatically proceeding calibration phase is arranged in the region of the electronic evaluation unit, characterised in that a calibrating device (25) arrangeable on the measuring head (12) is provided and comprises a calibrating cap (26) and guide means (35) for connecting the calibrating cap (26) to the sensors of the measuring head (12), and in that the calibrating cap (26) has an external gas connection (28) for a testing gas which flows through a cap-shaped housing to the measuring head (12), an electronic region (30) generating the calibrating signals to be picked up by the measuring head (12), a transmitter (36) which sends the calibrating signal to a receiving unit (D1, D2) in the measuring head (12), after which the main calibrating signal can be generated by the in situ electronic unit connected to the respective gas sensor of the measuring head (12) and transmitted to the electronic evaluation unit (10), as a result of which the automatically proceeding calibration phase can be initiated, and has a power supply.

2. Apparatus according to claim 1, characterised in that the calibrating signal is transmittable to the receiving unit (D1, D2) in the measuring head (12) when the calibrating cap (26) is in the calibrating position and the testing gas is supplied to the gas-sensitive element of each sensor.

3. Apparatus according to claim 1 or 2, characterised in that the calibrating cap (26) acts on the measuring head (12) and, in continuation thereof, the central electronic evaluation unit (10) connected thereto by means of a specific calibrating signal relating to the respective sensor (S1, S2, T) or a main calibrating signal.

4. Apparatus according to any one of claims 1 to 3, characterised in that the calibrating signal is an optical signal, e.g. an infrared signal, pulsed at a predetermined frequency, generated by a transmitting diode (36) and transmitted to a receiving diode (D1, D2) in the measuring head when a flow sensor in the calibrating cap (26) signals the presence of the testing gas, and a contact sensor (34) or a switch operated by the operator signals the mounting of the calibrating cap on the respective sensor to be calibrated.

5. Apparatus according to claim 4, characterised in that the flow sensor is an NTC resistor which is arranged in series with the contact sensor, see switching pin (34), signalling the mounting of the calibrating cap, and which activates the transmitting diode (36) for sending the calibrating signal.

6. Apparatus according to any one of claims 1 to 5, characterised in that, after receiving the calibrating signal and identifying the respective sensor, the central electronic evaluation unit (10) sends a calibration confirmation signal back to the respective measuring head (12), in that the measuring head (12) then sends a calibration confirmation signal to the operator and in that the central electronic evaluation unit automatically indicates the end of the calibration process by inducing the calibration confirmation signal while simultaneously adopting the new calibration value or values.

7. Apparatus according to claim 6, characterised in that, in the calibration phase, the central electronic evaluation unit (10) tests the measured value, received from the sensor being acted upon by the calibrating gas, for constant behaviour over time for a predetermined period and stores and adopts the new calibration values as a zero value for a measured value below a predetermined threshold, e.g. zero gas, or as a pitch value, e.g. sensitivity, of predetermined magnitude for a measured value above a predetermined threshold.

8. Apparatus according to any one of claims 1 to 7, characterised in that the central electronic evaluation unit (10) is connected to a plurality of measuring heads (12; 12a, 12b ... 12g) in situ solely via a single common two-wire line (11), in that the intelligence, or microprocessor, of each measuring head (12) makes bi-directional digital communication with the central electronic evaluation unit possible in situ and in that at least two sensors of different types or different measuring ranges and an additional temperature sensor are connected to each measuring head (12; 12a, 12b, 12c ... 12g).

9. Apparatus according to claim 8, characterised in that, in the connection between each respective sensor and measuring head, there is an identification means identifying the sensor according to type, measuring gas and measuring range, the identification being picked up by the measuring head.

10. Apparatus according to claim 9, characterised in that the sensors (13) to be connected to the measuring heads (12, 12a to 12g) each have standardised base connections, and the measuring heads have correspondingly formed receiving openings, e.g. a plug and socket, and in that the identification means provided on the respective sensor is recognisably in the form of electrical contacts for the measuring head.

11. Apparatus according to claim 10, characterised in that solder contact bridges for the measuring head and base mount are identifiably arranged on the respective sensor and indicate the type, measuring gas and measuring range thereof in binary coded form.

12. Apparatus according to any one of claims 8 to 11, characterised in that storage means, containing data associated with specific sensors according to type, measuring gas and measuring ranges, are provided at least in the region of the central electronic evaluation unit in such a manner that, after receiving the sensor identification, corresponding status signals, the respective measured values and the calibration values, the electronic evaluation unit can evaluate and process them.

13. Apparatus according to any one of claims 8 to 12, characterised in that corresponding operating routines for each received sensor identification are stored in the memory of the central electronic evaluation unit, the memory being formed as an EPROM.

## Revendications

1. Appareil pour calibrer automatiquement des détecteurs de gaz et destiné en particulier aux systèmes de détection de gaz comportant un certain nombre de têtes de mesure (12a, 12g) portant des détecteurs et raccordées électroniquement à une électronique centrale d'exploitation, ces têtes contenant une électronique décentralisée reliée par une ligne à grande distance (11) à l'électronique centrale d'exploitation (10), ainsi que des moyens pour amener au moins un gaz de contrôle dans la partie sensible au gaz de chaque détecteur, la tête (12) portant ce détecteur recevant un signal de calibrage lorsqu'on engage la phase de calibrage, tandis qu'au niveau de l'électronique d'exploitation il est prévu un organe de réglage pour recevoir les nouvelles valeurs étalonnées résultant de la phase de calibrage se déroulant automatiquement,
caractérisé en ce qu'
• il est prévu un dispositif de calibrage (25) pouvant être monté sur la tête de mesure (12), et comportant un chapeau de calibrage (26) équipé de moyens de guidage (35) pour le relier aux détecteurs de la tête (12),
• le chapeau de calibrage (26) est équipé d'un raccord de gaz (28) destiné au gaz de contrôle qui arrive à travers un boîtier en forme de capot à la tête de mesure (12), d'une électronique (30) produisant le signal que doit recevoir la tête de mesure (12), d'un émetteur (36) qui envoie le signal de calibrage à une unité réceptrice (D1, D2) logée dans la tête de mesure (12), de sorte que l'électronique décentralisée appartenant à la tête de mesure (12) et reliée au détecteur de gaz considéré peut générer le signal de calibrage à transmettre à l'unité de contrôle d'exploitation (10) et ainsi engager la phase de calibrage à déroulement automatique,
• le chapeau de calibrage est équipé d'une alimentation en courant.

2. Appareil selon la revendication 1,
caractérisé en ce que
le signal de calibrage peut être envoyé à l'unité réceptrice (D1, D2) logée dans la tête de mesure (12) dès que le chapeau de calibrage (26) se trouve dans la position d'étalonnage et que le gaz de contrôle est parvenu à l'élément sensible au gaz du détecteur considéré.

3. Appareil selon la revendication 1 ou 2,
caractérisé en ce que
le chapeau de calibrage (26) adresse à la tête de mesure (12) et par suite à l'électronique centrale d'exploitation (10) qui lui est reliée, un signal de calibrage spécifique au détecteur considéré (S1, S2, T) à savoir un signal principal de calibrage.

4. Appareil selon une des revendications 1 à 3,
caractérisé en ce que
le signal de calibrage est un signal optique pulsé à une fréquence donnée (signal infrarouge), produit par une diode émettrice (36) et envoyé à une diode réceptrice (D1, D2) située dans la tête de mesure lorsqu'un détecteur d'écoulement logé dans le chapeau de calibrage (26) signale la présence du gaz de contrôle et lorsqu'un détecteur de contact (34) ou un contacteur de l'installation actionné par la personne de service signalent l'installation de la tête sur le détecteur à calibrer.

5. Appareil selon la revendication 4,
caractérisé en ce que
le détecteur d'écoulement est une thermistance qui est montée en série avec le détecteur de contact ou une tige de contact (34) signalant l'installation de chapeau de calibrage et qui active la diode émettrice (36) pour qu'elle délivre le signal de calibrage.

6. Appareil selon une des revendications 1 à 5,
caractérisé en ce que
l'électronique centrale d'exploitation (10), après qu'elle ait reçu le signal de calibrage et reconnu le détecteur concerné, renvoie un signal de constatation de calibrage à la tête de mesure (12) qui transmet alors à la personne de service un signal de constatation de calibrage, l'électronique centrale d'exploitation indiquant la fin de l'opération de calibrage en agissant sur le signal de constatation de calibrage tout en prenant en compte simultanément la ou les nouvelles valeurs de calibrage.

7. Appareil selon la revendication 6,
caractérisé en ce que
l'électronique centrale d'exploitation (10) au cours de la phase de calibrage, contrôle pendant un intervalle de temps donné que la valeur mesurée provenant du détecteur concerné soumis à l'action de gaz étalon, a un comportement constant dans le temps, et met en mémoire et prend en charge les nouvelles valeurs d'étalonnage, dans le cas d'une valeur de mesure se trouvant en dessous d'un seuil donné, par exemple avec du gaz zéro comme valeur de zéro ou si elle se trouve au-dessus du seuil donné, comme valeur de pente, par exemple la sensibilité.

8. Appareil selon une des revendications 1 à 7,
caractérisé en ce que
l'électronique centrale d'exploitation (10) est reliée à une pluralité de têtes de mesure (12, 12a, 12b...12g) situées en un lieu donné, simplement par un conducteur commun à deux fils (11), l'intelligence, c'est-à-dire le microprocesseur de chaque tête de mesure (12) permettant sur place d'établir une communication numérique bidirectionnelle avec l'électronique centrale d'exploitation, tandis qu'à chaque tête de mesure (12, 12a, 12b....12g) sont raccordés au moins deux détecteurs de genres différents ou ayant des plages de mesure différentes, ainsi qu'un détecteur supplémentaire de température.

9. Appareil selon la revendication 8,
caractérisé en ce que
dans la liaison entre chaque détecteur et la tête de mesure s'effectue une reconnaissance identifiant le détecteur par son type, le gaz qu'il mesure et sa plage de mesure, cette identification étant saisie par la tête de mesure.

10. Appareil selon la revendication 9,
caractérisé en ce que
les détecteurs (13) à relier aux têtes de mesure (12, 12a à 12g) comportent chacun un culot de raccordement uniformisé tandis que les têtes de mesure comportent des ouvertures de réception correspondantes, par exemple des broches et des douilles, la caractérisation attribuée à chaque détecteur étant matérialisée de manière visible par les contacts électriques destinés à la tête de mesure.

11. Appareil selon la revendication 10,
caractérisé en ce que
chaque détecteur porte des ponts entre les contacts soudés donnant, sous forme binaire codée, le type de gaz de mesure et la plage de mesure, de manière à être identifiable pour la prise du socle de la tête de mesure.

12. Appareil selon une des revendications 8 à 11,
caractérisé en ce qu'
il est prévu, au moins au niveau de l'électronique centrale d'exploitation, des moyens mettant en mémoire, pour chaque détecteur, des données concernant son type, le gaz qu'il mesure et ses plages de mesure, de manière que l'électronique centrale, après réception de l'identification du détecteur, peut valoriser et exploiter les signaux correspondants à son état, les valeurs mesurées et les valeurs de calibrage.

13. Appareil selon une des revendications 8 à 12,
caractérisé en ce que
la mémoire de l'électronique centrale d'exploitation, de type EPROM, contient les routines de travail correspondant à chaque identification de détecteur reçue.
